Europäisches Patentamt

European Patent Office

Office européen des brevets

(11) Veröffentlichungsnummer: **0 319 762**
**A1**

## (12) EUROPÄISCHE PATENTANMELDUNG

(21) Anmeldenummer: 88119362.7

(22) Anmeldetag: 22.11.88

(51) Int. Cl.4: **A61B 1/12 , G07C 3/00**

(30) Priorität: 10.12.87 DE 3741833

(43) Veröffentlichungstag der Anmeldung:
14.06.89 Patentblatt 89/24

(84) Benannte Vertragsstaaten:
**BE CH DE FR GB LI**

(71) Anmelder: **Richard Wolf GmbH**
**Pforzheimer Strasse 32**
**D-7134 Knittlingen(DE)**

(72) Erfinder: **Ams, Felix, Dipl.-Ing.**
**Gründle Strasse 5**
**D-7539 Kämpfelbach(DE)**

(74) Vertreter: **Wilcken, Hugo, Dr. et al**
**Patentanwälte Dr. Hugo Wilcken Dipl.-Ing.**
**Thomas Wilcken Musterbahn 1**
**D-2400 Lübeck(DE)**

(54) **Einrichtung zur Überwachung der Betriebzustände von Versorgungsgeräten von Endoskopen.**

(57) Einrichtung zur Überwachung der Betriebszustände von Versorgungsgeräten (3,4) von Endoskopen mit einem Prozessor (2), der einen Datenspreicher (2a) aufweist, welcher über Schnittstellen (10) mit den genannten Versorgungsgeräten (3,4) verbunden ist, bei der der Prozessor (2) die Betriebszustände der Versorgungsgeräte abfragt, sie mit in seinem Datenspreicher (2a) abgespeicherten Soll-Werten vergleicht und das Ergebnis des Vergleichs auf einer oder mehreren Anzeigevorrichtungen (7,9) zur Anzeige bringt.

EP 0 319 762 A1

## Einrichtung zur Überwachung der Betriebszustände von Versorgungsgeräten von Endoskopen

Die Erfindung geht von einer Einrichtung gemäß dem Oberbegriff des Anspruches 1 aus.

Es ist bekannt (DE-PS 36 01 395), bei Endoskopwaschmaschinen eine Anzeige für anormale Betriebszustände vorzusehen, die sich durch Mittel zum Feststellen des Flüssigkeitspegels in einem Speisewasserbehälter, im Desinfektionslösungsbehälter und im Waschbehälter kennzeichnen.

Demgegenüber handelt es sich nach der Erfindung um ein Endoskop, welches von mindestens einem Peripheriegerät versorgt wird. Derartige Versorgungsgeräte können beispielsweise Lichtprojektoren, Luftpumpen zur Erzeugung eines Luftstromes zum Freispülen des distalen Endoskopfensters, Pumpen für Spülflüssigkeit, HF-Generatoren und dergleichen sein.

Der Erfindung liegt die Aufgabe zugrunde, eine Einrichtung der eingangs genannten Art zu schaffen, welche die Betriebszustände der genannten Versorgungsgeräte überwacht und Fehler und/oder die Betriebszustände zur Anzeige bringt.

Dabei sind unter dem Begriff "Betriebszustände" Größen und Zustände zu verstehen, durch welche die momentan aktuellen Betriebsparameter gekennzeichnet sind. Dies sind bei Lichtprojektoren beispielsweise die Lampenspannung, Lampenstrom, Arbeitstemperatur, Blitzfrequenz bei Erzeugung von Stroboskoplicht und dergleichen.

Die Aufgabe wird gelöst durch die kennzeichnenden Merkmale des Anspruchs 1. Vorteilhafte Weiterbildungen ergeben sich aus den Unteransprüchen.

Durch die erfindungsgemäße Einrichtung wird erreicht, daß dem Bedienungspersonal des Endoskops die für dessen Betrieb wesentlichen Informationen in Form von angezeigten Ist-Zuständen der Betriebsparameter und/oder von Fehleranzeigen bei Abweichungen der Ist-Werte von vorgegebenen Soll-Werten zur Verfügung stehen.

Die Weiterbildung des Endoskops gemäß Anspruch 6 bietet darüber hinaus den Vorteil, daß neben der Anzeige von Informationen eine Regelung von Betriebsparametern vorgenommen werden kann.

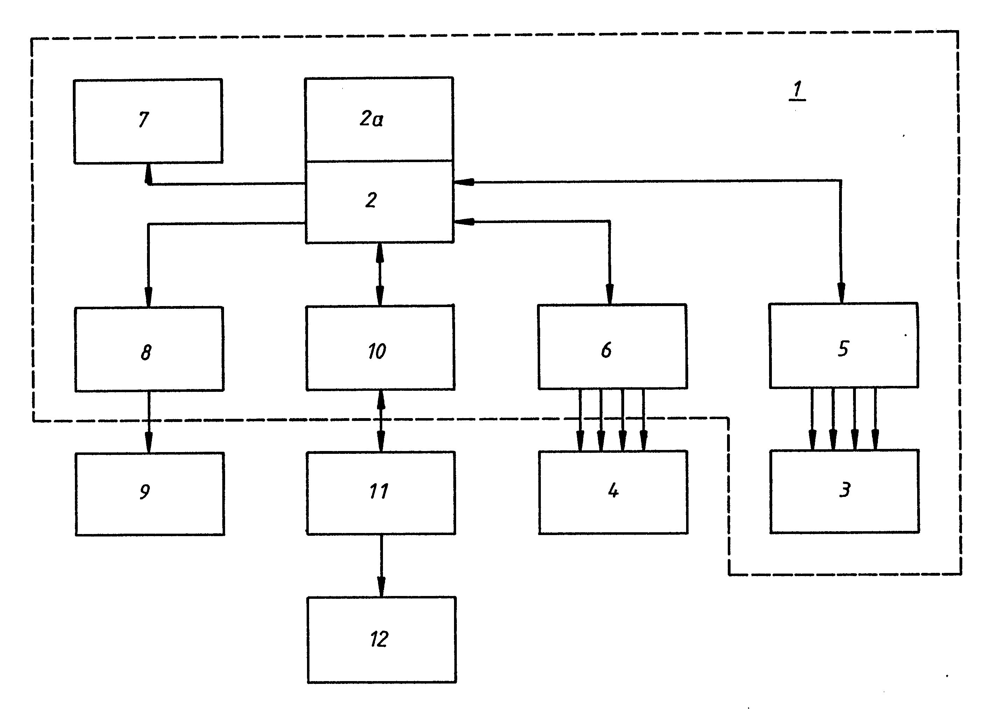

Die Erfindung wird anhand eines Ausführungsbeispieles gemäß der Zeichnung näher erläutert. Sie zeigt ein Blockschaltbild der erfindungsgemäßen Einrichtung 1 mit einem Mikroprozessor 2, der über einen Programm- und einem Datenspeicher verfügt. Der Programmspeicher 2a speichert in bekannter Weise ein vorgegebenes Programm, nach dem die Betriebszustände der einzelnen Versorgungsgeräte 3,4 abgefragt werden. Die Versorgungsgeräte 3,4 sind über paralelle Schnittstellen 5,6 mit dem Prozessor 2 verbunden.

Im gezeigten Ausführungsbeispiel ist als Anzeigevorrichtung für die vom Prozessor 2 erzeugten Ausgangssignale eine Klartextanzeige 7 sowie ein über ein Video-Interface 8 angeschlossener Monitor 9 vorgesehen, wobei das Video-Interface aus den Ausgangssignalen des Prozessor 2 fernsehkompatible Signale aufbereitet.

Darüberhinaus ist ein Rechner 11 mit Drucker 12 über eine vorzugsweise serielle Schnittstelle 10 am Prozessor 2 angeschlossen.

Als von der Einrichtung zu überwachende Betriebszustände sind beispielsweise zu nennen bei einem Versorgungsgerät

a) Lichtprojektor: Lampenspannung, Lampenstrom, Arbeitstemperatur, Blitzfrequenz bei Erzeugung von Stroboskoplicht, Ausfall der Lampe, Lichtregelung automatisch oder manuell und dergleichen,

b) Pumpe: Fördermenge, Druck der Spülflüssigkeit im Bereich der Pumpe oder im Bereich der Körperhöhle, Überdruck und dergleichen.

Bei der Verwendung eines Lüfters als Versorgungsgerät kann die Drehzahl überwacht werden, ebenso wie bei einem Werkzeug zum Abtragen von Knorpelgewebe bei der Arthroskopie.

Es wird an dieser Stelle ausdrücklich erwähnt, daß die obige Auflistung von Versorgungsgeräten nicht abschließend ist.

Die Betriebszustände der Vorsorgungsgeräte 3,4 werden, soweit sie nicht elektrischer Natur sind und direkt dem Prozessor 2 zugeführt werden können, von Gebern erfaßt und in elektrische Signale umgewandelt.

Der Prozessor 2 fragt nach dem vorgegebenen Programm über die parallelen Schnittstellen 5,6 die jeweiligen Betriebszustände ab und vergleicht die Werte mit den in seinem Datenspeicher abgespeicherten Soll-Werten. Weichen die Ist-Werte von den Soll-Werten ab, kann die Abweichung und/oder eine Fehlermeldung als schriftlicher Hinweis auf dem Monitor 9 oder der Klartextanzeige 7 angezeigt werden.

Darüberhinaus ist es möglich, daß der korrekte Sitz eines extern an ein Versorgungsgerät, beispielsweise an einem Lichtprojektor angeschlossene Stecker (nicht dargestellt), mittels eines durch den Stecker betätigten Schalters, Lichtschranke oder dergleichen überwacht werden.

Die serielle Schnittstelle 10 dient dazu, Daten vom Prozessor 2 zum externen Rechner 11 zu übertragen. Der Rechner 11 kann die erfaßten Betriebszustände der Versorgungsgeräte 3,4 weiterverarbeiten und auf den Drucker 12 beispielsweise als Graphik darstellen.

Im Datenspeicher des Prozessors 2 kann neben den genannten Soll-Werten auch eine vorgegebene Lebensdauer der Lampe eingespeichert werden. Während des Betriebes der Lampe wird dann die Betriebsdauer mittels eines elektronischen Betriebsstundenzählers ermittelt und mit dem eingegebenen Wert für die Lebensdauer der Lampe verglichen. Nähert sich die Betriebsdauer der vorgesehenen Lebensdauer,kann der Prozessor 2 ein dementsprechendes Signal ("Lampenwechsel" o.dgl.) zur Anzeige bringen.

Neben den erwähnten Vorteilen besteht bei der vorgeschlagenen Einrichtung die Möglichkeit, nach dem Einschalten einen Selbsttestzyklus zu fahren,um so das Bedienungspersonal zuverlässig über den Zustand der Einrichtung zu informieren. Dazu fragt der Prozessor 2 direkt nach dem Einschalten die für die Betriebsbereitschaft notwendigen Parameter (beispielsweise Lampe in Ordnung, Steckverbindung korrekt) ab und bringt etwaige Fehler zur Anzeige.

Der Prozessor 2 kann neben den genannten Aufgaben gemäß einer weiteren vorteilhaften Ausbildung der Einrichtung auch Regelfunktionen übernehmen, beispielsweise für die Drehzahl eines Lampenlüfters, wenn diese bei zu hoher Betriebstemperatur erhöht werden muß.

Schließlich braucht die Anzeige nicht nur optisch zu erfolgen. Denkbar ist auch eine akustische Anzeige, etwa wenn ein Fehler beim Selbsttest der Einrichtung festgestellt worden ist.

Es ist auch möglich, nicht nur bei fehlerhaftem Betrieb eine Anzeige auf den Anzeigvorrichtungen zu generieren, sondern auch bei fehlerfreiem Betrieb.

## Ansprüche

1. Einrichtung zum Überwachen der Betriebszustände von Versorgungsgeräten von Endoskopen, dadurch gekennzeichnet, daß ein Prozessor (2) vorgesehen ist, der einen Datenspeicher (2a) aufweist, welcher über Schnittstellen (5,6) mit den jeweiligen Versorgungsgeräten (3,4) verbunden ist, und daß der Prozessor (2) die Betriebszustände des Versorgungsgerätes (3,4) abfragt, sie mit in seinem Datenspeicher abgespeicherten Soll-Werten vergleicht und das Ergebnis des Vergleichs auf einer oder mehreren Anzeigevorrichtungen (7,9) direkt oder in beliebiger graphischer Darstellung anzeigt.

2. Einrichtung nach Anspruch 1, dadurch gekennzeichnet, daß der Prozessor (2) über eine weitere Schnittstelle (10) mit einem Rechner (11) mit Drucker (12) verbunden ist.

3. Einrichtung nach Anspruch 1 oder 2, dadurch gekennzeichnet, daß eine Anzeigevorrichtung ein Monitor (9) ist.

4. Einrichtung nach einem der Ansprüche 1 bis 3, dadurch gekennzeichnet, daß eine Anzeigevorrichtung eine Klartextanzeige (7) ist.

5. Einrichtung nach einem der Ansprüche 1 bis 4, dadurch gekennzeichnet, daß der Prozessor (2) zusätzlich die korrekte Verbindung eines extern an einem Versorgungsgerät angeschlossenen Steckers überwacht.

6. Einrichtung nach einem der Ansprüche 1 bis 5, dadurch gekennzeichnet, daß der Prozessor (2) Betriebsparameter von Versorgungsgeräten (3,4) in Abhängigkeit vorwählbarer Sollwerte regelt.

EP 0 319 762 A1

| Europäisches Patentamt | EUROPÄISCHER RECHERCHENBERICHT | Nummer der Anmeldung |
|---|---|---|
| | | EP 88 11 9362 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 064 818 (DAVY-LOEWY LTD) <br> * Seite 2, Zeilen 85-119 * | 1,3,6 | A 61 B 1/12 <br> G 07 C 3/00 |
| A | | 4 | |
| | --- | | |
| X | DE-A-2 658 818 (BEA BAUGESELLSCHAFT FUER ELEKTRISCHE ANLAGEN AG) <br> * Seite 7, Zeile 7-Seite 8, Zeile2, Figur 1 * | 1,2 | |
| | --- | | |
| X | DE-A-3 406 128 (TOSHIBA KIKAI K.K.) <br> * Seite 9, Zeile 1-Seite 14, Zeile 18; Figuren 1,4 * | 1,3 | |
| | ----- | | |

|  | RECHERCHIERTE SACHGEBIETE (Int. Cl.4) |
|---|---|
| | A 61 B 1/12 <br> G 07 C 3/00 |

Der vorliegende Recherchenbericht wurde für alle Patentansprüche erstellt

| Recherchenort | Abschlußdatum der Recherche | Prüfer |
|---|---|---|
| BERLIN | 17-02-1989 | WEIHS J.A. |